# EUROPEAN PATENT APPLICATION

(11) **EP 2 246 050 A1**
(43) Date of publication of application: **03.11.2010**
(21) Application number: 09158880.6
(22) Date of filing: 27.04.2009
(51) Int. Cl.: A61K 31/381, A61K 31/4535, A61H 1/00, A61P 19/00

(54) **Combination therapy comprising mechanical vibration for the treatment of a disease of the musculoskeletal or nervous system**

(71) Applicant: IMR - International Medical Research - Partner GmbH, 82166 Gräfelfing (DE)
(72) Inventor: Rother, Ilka, 82166, Gräfelfing (DE); Rother, Matthias, 82166, Gräfelfing (DE); Seidel, Egbert, 99428, Weimar-Tröbsdorf (DE)
(74) Representative: Maiwald, Walter

(57) **Abstract**

The present invention pertains to the treatment of diseases of the musculoskeletal system and/or diseases of the peripheral nervous system, particularly by improvement of bone mineral density, joint cartilage, muscle strength and/or peripheral neuronal function. The present invention provides a method for enhancing the therapeutic or preventive effect of a pharmaceutically active ingredient on the musculoskeletal system and/or peripheral nervous system. Said method is a combination therapy which combines the administration of a pharmaceutically active ingredient with the application of mechanical vibration treatment to a vertebrate. The combination of the mechanical vibration treatment with the administration of the pharmaceutically active ingredient improves the therapeutic effect of the latter. Present invention thus also pertains to a pharmaceutically active ingredient for use in a combination therapy in combination with mechanical vibration treatment for the prevention, delay of progress, or treatment of a disease of the musculoskeletal system and/or a disease of the peripheral nervous system in a vertebrate.

## Description

### Field of the Invention

The present invention pertains to the treatment of diseases of the musculoskeletal system and/or diseases of the peripheral nervous system, particularly by improvement of bone mineral density, joint cartilage, muscle strength and/or peripheral neuronal function. The present invention provides a method for enhancing the therapeutic or preventive effect of a pharmaceutically active ingredient on the musculoskeletal system and/or peripheral nervous system. Said method is a combination therapy which combines the administration of a pharmaceutically active ingredient with the application of mechanical vibration treatment to a vertebrate. The combination of the mechanical vibration treatment with the administration of the pharmaceutically active ingredient improves the therapeutic effect of the latter. Present invention thus also pertains to a pharmaceutically active ingredient for use in a combination therapy in combination with mechanical vibration treatment for the prevention, delay of progress, or treatment of a disease of the musculoskeletal system and/or a disease of the peripheral nervous system in a vertebrate.

### Background of the Invention

Diseases of the musculoskeletal system and/or diseases of the peripheral nervous system are a leading cause of disability and account for many chronic diseases in developed countries. Typical diseases of the musculoskeletal system are degenerative bone diseases like osteoporosis, degenerative joint diseases like osteoarthritis, fasciitis, degenerative cartilage diseases, jaw diseases, muscular diseases (of skeletal or smooth muscle) like atrophy or dystrophy, musculoskeletal abnormalities, rheumatic diseases, tendinitis, soft tissue injuries and tennis elbow. Two of the most prevalent diseases of the musculoskeletal system are osteoporosis and osteoarthritis. Typical diseases of the peripheral nervous system are peripheral neuropathies and neurocutaneous disorders. Peripheral nerve disorders may be divided into hereditary and acquired forms. The most common hereditary form is Charcot-Mario-Tooth syndrome. The most common acquired forms are neuropathies associated with diabetes mellitus and alcoholism.

Osteoporosis is a disease of the musculoskeletal system, belonging to the group of degenerative bone diseases, characterized by low bone mass and deterioration of bone tissue, thus leading to an increased risk of fracture. In osteoporosis the bone mineral density (BMD) is reduced, bone microarchitecture disrupted, and the amount and variety of non-collagenous proteins in bone is altered. Due to the risk of fragility fractures, osteoporosis may significantly affect life expectancy and quality of life, becoming an increasingly public health problem. Furthermore, osteoporosis is most common in post-menopausal women over the age 50. Following the menopause women experience a rapid bone loss due to the decrease in estrogen production, which plays a major role in regulation of the bone formation-resorption equilibrium in the body.

The diagnosis of osteoporosis is made on measuring the bone mineral density. The most popular method is dual energy X-ray absorptiometry. Furthermore, blood tests and X-rays provide additional information for the diagnosis.

Several medications are known to treat degenerative bone diseases depending on gender, age, body weight, mode of action, as well as stage of the degenerative bone disease. Medication including calcium, vitamin D, and fluoride supplements, the group of bisphosphonates, hormonal agents, dual action bone agents, and selective estrogen receptor modulators are currently used for preventing and treating degenerative bone diseases, like osteoporosis, osteitis deformans (Paget's disease), bone metastasis and other conditions that feature bone fragility.

Pharmaceutical interventions in osteoporosis are differentiated into (i) antiresorptive therapy which inhibits bone resorption (bisphosphonates, selective estrogen receptor modulators (SERMs), estrogens) and (ii) anabolic or partly anabolic therapy (strontium ranelate, peptides of the parathyroid hormone family).

In confirmed osteoporosis, bisphosphonate drugs are the first-line treatment in post-menopausal women. Bisphosphonates inhibit osteoclast action and the resorption of bone by blocking the enzyme farnesyl diphosphate synthase (FPPS) in the mevalonate pathway, causing prenylation. One of the disadvantages of the orally taken bisphosphonates is that they are relatively poorly absorbed, and must therefore be taken on an empty stomach, with no food or drink to follow for the next 30 minutes. Side-effects include occurrence of an upset stomach, inflammation, esophagitis, as well as osteonecrosis of the jaw.

Oral strontium ranelate is an alternative oral treatment, belonging to a class of drugs called "dual action bone agents" (DABAs), as it both increases deposition of new bone by osteoblasts and reduces the resorption of bone by osteoclasts. Strontium ranelate stimulates the calcium sensing receptors and leads to the differentiation of pre-osteoblasts to osteoblasts, which increases the bone formation. Strontium ranelate is indicated for the treatment of post-menopausal osteoporosis to reduce the risk of vertebral and hip fractures, and is taken as 2 g one sachet once a day. The most common side-effects observed are nausea, diarrhea, headache or eczema.

Hormonal treatment of degenerative bone diseases using peptides of the parathyroid hormone family is an alternative treatment for patients who cannot tolerate orally given medicaments. Teriparatide is injected subcutaneously into thigh or abdominal wall. It acts like parathyroid hormones by stimulating osteoblasts, resulting in increased bone mineral density. WO 2006/015005 describes the use of a thyroid stimulating hormone (TSH; thyrotropin) in the treatment of degenerative bone disorders.

Furthermore, hormone replacement therapy (HRT) using estrogens is an effective treatment of osteoporosis, especially in postmenopausal women. Side-effects may include headache, upset stomach, stomach cramps or bloating, diarrhea, swelling, acne, pain, severe mental depression, fever, and tiredness.

Raloxifene is an oral selective estrogen receptor modulator (SERM) that has estrogenic effects on bone. Estrogen plays a major role in regulation of the bone formation-resorption equilibrium in the body, regulating the bone mineral density by stimulating the osteoblast activity, therefore balancing the osteoblast and osteoclast activity in the trabecular bone. Raloxifene is indicated for the prevention and treatment of osteoporosis in post-menopausal women, and for reduction in risk of invasive breast cancer in postmenopausal women with osteoporosis. Common adverse events considered to be drug-related are hot flashes and leg cramps. The active pharmaceutical ingredient is in the form of raloxifene-HCl and may infrequently also cause serious blood clots to form in the legs, lungs, or eyes. Other reactions experienced are leg swelling, leg pain, trouble breathing, chest pain, or vision changes. Raloxifene is available as white, oval tablets (60 mg). The recommended dose for adults and the elderly is one tablet taken once a day, with or without food. Raloxifene is intended for long-term use. Other SERMs in the late clinical development phases are lasofoxifene, arzoxifene and bazedoxifene.

Another way of differentiating pharmaceutical intervention in osteoporosis is a differentiation into (i) products that inhibit cell activity or cause cell death of target cells (like bisphosphonates) and (ii) products that interact with a living cell or cellular components like receptors, thus modulating their activity (like SERMs, DABAs and peptides of the parathyroid hormone family).

Another important disease of the musculoskeletal system is osteoarthritis, belonging to the group of degenerative joint diseases. Osteoarthritis usually involves degeneration of the joint cartilage and/or deformation of joint parts and is another leading cause of disability in elderly people. Treatment of osteoarthritis is separated into symptomatic treatment - predominantly treatment of pain and inflammation related to osteoarthritis - and disease modifying treatment aiming at inhibition of cartilage degeneration. Typical examples of symptomatic treatment are analgesic medication including paracetamol, NSAIDs, steroids, opiates and other analgesics. Disease modifying agents include hyaluronic acid and its derivatives, glucosamines, chondroitin, diacerine, or a pharmaceutically acceptable salt thereof.

A further important disease of the musculoskeletal system is muscular atrophy, belonging to the group of muscular diseases, characterized by loss of muscle tissue. In muscular atrophy the decrease in the mass of the muscle can progress partially or completely. Pharmacological treatment involves androgenic compounds, such as testosterone which modulates the activity of the androgen receptor, and/or a growth hormone which modulates the activity of a growth hormone receptor. Typically, androgenic compounds belong to the group of selective androgen receptor modulators.

Peripheral neuropathies are diseases of the peripheral nervous system resulting from diffuse lesions of peripheral nerves, characterized by weakness, pain, sensory loss and autonomic dysfunction. The disease and treatment effects can be characterized by evaluating the symptoms like pain, sensory function using electrophysiological methods like evaluation of nerve conduction velocity. Treatment of peripheral neuropathies is focused on eliminating the causal factor leading to acquired peripheral neuropathy but also symptomatic treatment of abnormal nerve function like sensory loss. Symptomatic treatment acts on various ion channels and/or receptors of a sensory neuron, like a Na⁺ channel, a GABA mediated receptor and/or a proprioceptor, of the nerve cell to inhibit neuronal excitability. Drugs used with this intention include antiepileptics like gabapentin and carbamazepine, and local anaesthetics like lidocaine. Symptomatic treatment would also be possible by increasing the sensitivity of neural sensors, like the proprioceptors, but up to now there is no good pharmacological approach available for such treatment.

A method of restoring or remodeling, rather than disrupting, the normal processes and/or structures of bone, cartilage and musculoskeletal structure represents a good alternative method for the prevention, delay of progress, or treatment of a disease of the musculoskeletal system, such as osteoporosis or osteoarthritis. This alternative method is not based on drugs, but on mechanical vibration, in particular, whole-body vibration (WBV).

Whole-body vibration therapy is a safe and widely used mechanical vibration treatment of a disease of the musculoskeletal system, especially of muscular disorders and osteoporosis. Whole-body vibration is a neuromuscular training method that has shown a positive effect on bone mineral density, as well as decrease in pain associated with osteoporosis.

Another immediate effect of whole-body vibration is an improvement of circulation, as the rapid contraction and relaxation of the muscles basically work as a pump on the blood and lymphatic vessels, increasing the speed of the blood flow through the body. This allows a better and faster supply with nutrients, thus helping to increase the bone mineral density.

The Berlin Bedrest Study (BBR) in 2004 proved that 10 minutes of vibration training six times a week prevented muscle and bone loss in total bedrest over 55 days. Rubin et al. (Age and Ageing, 2006, 35-52:ii32-ii36) investigated low-level mechanical signals and their potential as non-pharmacological intervention for osteoporosis and came to the conclusion that these signals can increase cancellous bone volume fraction, trabecular thickness, trabecular number and enhance bone stiffness and strength. Ruan et al. (Chinese Medical Journal, 2008, 121(12):1155-1158) demonstrated that vibration therapy reduces chronic back pain and increases the femoral neck and lumbar bone mineral density in postmenopausal women with osteoporosis. Verschueren et al. (Journal of Bone and Mineral Research, 2004, 19(3):352-359) demonstrated the positive effect of whole-body vibration training on hip density, muscle strength and postural control in postmenopausal women.

CN 101224159 discloses a vibration motion instrument for the prevention and treatment of osteoporosis. It indicates the problem that patients may adapt to the vibration therapy during long-term treatments, hence lowering its beneficial effect.

There still exists a need for the development of new therapeutic methods for preventing, delaying or treating diseases of the musculoskeletal system and/or diseases of the peripheral nervous system. Besides the need to improve efficacy, there is also a need for new therapeutic methods reducing the severe side-effects associated with currently used medicaments at their currently recommended dosages for the treatment of diseases of the musculoskeletal system and/or diseases of the peripheral nervous system. As the substantial side-effects observed can lead to polypharmacy, and medicaments often have to be used over a long-term period to be effective, new methods for the reduction of said drawbacks of existing methods are also to be sought. Furthermore, reducing the number and/or amount of the APIs administrated to a subject is especially desirable to avoid polypharmacy, drug-drug interactions and other side-effects, in particular for elderly people. The development of new therapeutic methods for osteoporosis, osteoarthritis, muscular atrophy and acquired peripheral neuropathy, in particular for osteoporosis, is one specific objective of present invention.

These and other objectives as they will become apparent from the ensuing description of the invention are solved by the present invention as described in the independent claims. The dependent claims pertain to preferred embodiments.

### Summary of the Invention

The present invention provides a method for enhancing the therapeutic or preventive effect of a pharmaceutically active ingredient on a disease of the musculoskeletal system and/or a disease of the peripheral nervous system. Said method is a combination therapy which combines the administration of a pharmaceutically active ingredient with the application of mechanical vibration treatment to a vertebrate. The combination of the mechanical vibration treatment with the administration of the pharmaceutically active ingredient improves the therapeutic effect of the latter.

Said combination therapy typically comprises administering a pharmaceutically active ingredient which modifies the activity of a cell and/or a cellular component, particularly a receptor, and performing mechanical vibration treatment, in order to enhance the cell response to the pharmaceutically active ingredient. The combination of a pharmaceutically active ingredient with mechanical vibration treatment benefits from its potential synergistic effect, and may result in the increase of therapeutic effect, reduction of the amount of the pharmaceutically active ingredient used, reduction of side-effects and/or of polypharmacy.

In a specific aspect of the combination therapy according to the present invention, whole-body vibration stimulates body cells upon a cellular level, e.g. by increasing the responsiveness. Thus, present invention allows the use of fewer amounts of drugs in the treatment of a disease of the musculoskeletal system and/or a disease of the peripheral nervous system. In addition, the side-effects associated with the conventional methods of treatment of said disease may be reduced.

The combination therapy according to present invention is characterized by two elements: (A) the mechanical vibration treatment; and (B) the pharmaceutically active ingredient.

Present invention thus also pertains to a pharmaceutically active ingredient for use in a combination therapy in combination with mechanical vibration treatment for the prevention, delay of progress, or treatment of a disease of the musculoskeletal system and/or a disease of the peripheral nervous system in a vertebrate, preferably a disease of the musculoskeletal system.

In a first embodiment, present invention pertains to the treatment of a degenerative bone disease, particularly to the treatment of osteoporosis and/or to the improvement of bone mineral density. In said embodiment, the pharmaceutically active ingredient preferably is a compound which modifies the activity of an estrogen receptor and/or a calcium sensing receptor.

In a second embodiment, present invention pertains to the treatment of a degenerative joint disease, particularly to the treatment of osteoarthritis. In said embodiment, the pharmaceutically active ingredient preferably is a compound which modifies the activity of a chondrocyte (like glucosamine sulfate) and/or a subchondral osteocyte (i.e. an osteocyte of the subchondral bone).

In a third embodiment, present invention pertains to the treatment of a muscular disease, particularly to the treatment of muscular atrophy. In said embodiment, the pharmaceutically active ingredient preferably is a compound which modifies the activity of a muscle cell, and in particular the activity of an androgen receptor and/or a growth hormone receptor. Compounds that can be used for this purpose are androgens like SARMs, especially testosterone and growth hormones.

In a fourth embodiment, present invention pertains to the treatment of a disease of the peripheral nervous system, particularly to the treatment of acquired peripheral neuropathy. In said embodiment, the pharmaceutically active ingredient preferably is a compound which modifies the activity of ion channels, sensors and/or receptors of the nerve cell, like a Na⁺ channel, a GABA mediated receptor and/or a proprioceptor, in order to modify neuronal excitability.

In particular, the present invention provides the following preferred embodiments:
(1) a pharmaceutically active ingredient for use in a combination therapy in combination with mechanical vibration treatment for the prevention, delay of progress, or treatment of a disease of the musculoskeletal system and/or a disease of the peripheral nervous system in a vertebrate;
(2) the pharmaceutically active ingredient (1), for use in the combination therapy of a degenerative bone disease, in particular of osteoporosis;
(3) the pharmaceutically active ingredient according to (2) above, which modulates the activity of an estrogen receptor and/or a calcium sensing receptor;
(4) the pharmaceutically active ingredient (1), for use in the combination therapy of a degenerative joint disease, in particular of osteoarthritis;
(5) the pharmaceutically active ingredient according to (4) above, which modulates the activity of a chondrocyte and/or a subchondral osteocyte, and preferably modulates the activity of a glucose transporter of the chondrocyte;
(6) the pharmaceutically active ingredient (1), for use in the combination therapy of a muscular disease, in particular of muscular atrophy;
(7) the pharmaceutically active ingredient according to (6) above, which modulates the activity of an androgen receptor and/or a growth hormone receptor;
(8) the pharmaceutically active ingredient (1), for use in the combination therapy of a disease of the peripheral nervous system, in particular of acquired peripheral neuropathy;
(9) the pharmaceutically active ingredient according to (8) above, which modulates the activity of an ion channel, a sensor and/or a receptor of a nerve cell, in particular, of a Na⁺ channel, a GABA mediated receptor and/or a proprioceptor;
(10) a method for enhancing the therapeutic or preventive effect of a pharmaceutically active ingredient on a disease of the musculoskeletal system and/or a disease of the peripheral nervous system, comprising the application of mechanical vibration treatment to a vertebrate suffering from said disease; and
(11) the pharmaceutically active ingredient according to any one of (1) to (9) above, wherein the effective daily dose of said ingredient is lower than the respective daily dose of same effectiveness in the absence of mechanical vibration treatment.

The above embodiments (2) and (3) are presently particularly preferred.

### Detailed Description

The following terms and definitions will be used in the context of the present invention:
As used in the context of present invention, the singular forms of "a" and "an" also include the respective plurals unless the context clearly dictates otherwise. Thus, the term "a" or "an" is intended to mean "one or more" or "at least one", unless indicated otherwise.

The term "about" in context with a numerical value or parameter range denotes an interval of accuracy that the person skilled in the art will understand to still ensure the technical effect of the feature in question. The term typically indicates deviation from the indicated numerical value of +/- 10%, preferably +/- 5%.

"Comprising" in the context of present invention means that the components listed after the word "comprising" are either the sole components forming the combination (i.e. the combination consists of said components), or that in addition to said components at least one further component is present. In a preferred meaning, it means "consisting essentially of" (i.e. there may be further components, but of little or no technical relevance), and in its narrowest meaning, it is synonymous to "consisting of'.

The term "pharmaceutical composition" designates either a medicament or a composition suitable for preparation of a medicament. Typically, the term designates a medicament.

The term "pharmaceutically active ingredient" is synonymous to "active pharmaceutical ingredient" (API) and designates a pharmaceutically active compound or mixture of compounds. In a preferred aspect, it designates a single pharmaceutically active compound.

"Responsiveness" according to present invention designates the sensitivity of a cell or cellular component like a receptor, a sensor and/or an ion channel to an external stimulus, especially to the pharmaceutically active ingredient which is an element of the combination therapy of present invention. It can be determined in qualitative and quantitative features. E.g., when the speed and/or intensity of a cellular response to the API is increased (in comparison to administration of the API without concomitant mechanical vibration treatment), the responsiveness of the cell is enhanced. Vice versa, the responsiveness is reduced if the cellular response is delayed or suppressed. Methods for determination of cellular responses are well known to a person skilled in the art and are described in e.g. Vogel H.G., Vogel W.H. (eds.) Drug discovery and evaluation - pharmacological assays, Springer Verlag, Germany.

When a cell or cellular component is "stimulated" by mechanical vibration treatment in the context of present invention, this means that its responsiveness is enhanced.

When the activity of a cell or cellular component is modulated by a pharmaceutically active ingredient in the context of present invention, this means that the biological function of said cell or cellular component is altered by the API. This alteration of the biological function generally and preferably leads to the desired therapeutic effect of the API. Typically, activation of a cell or cellular component by an API is consistent with the known mode of action of said API as described, e.g., in the Product Information Leaflet or by the manufacturer's instructions, and the desired therapeutic effect is the known therapeutic effect of the API, e.g. as per said product information.

A "disease of the musculoskeletal system" is any disease of the muscles, their associated ligaments, other connective tissue, the bones and/or cartilage. Typical diseases of the musculoskeletal system are bone diseases, cartilage diseases, fasciitis, joint diseases, jaw diseases, muscular diseases (of skeletal or smooth muscle), musculoskeletal abnormalities, rheumatic diseases, tendinitis, soft tissue injuries and tennis elbow. In the context of present invention, a disease of the musculoskeletal system is preferably a degenerative bone disease, a degenerative joint disease, or a muscular disease. Of these, degenerative bone or joint diseases are preferred, degenerative bone diseases are more preferred, osteoporosis and osteoarthritis are particularly preferred, and osteoporosis is most preferred.

A "degenerative bone disease" in the context of present invention is any disease caused by or resulting in bone degeneration, preferably any disease resulting in bone degeneration. Such disease is typically characterized by bone density and/or bone mineral density (BMD) reduction, and/or loss of bone mass. The preferred bone degenerative disease in the context of present invention is osteoporosis.

A "degenerative joint disease" in the context of present invention is any disease characterized by cartilage loss and/or abnormalities of subchondral bone structures. The preferred degenerative joint disease in the context of present invention is osteoarthritis (synonym: *osteoarthritis deformans*).

A "muscular disease" in the context of present invention is any disease characterized by loss of muscle tissue. To the group of muscular diseases belong muscular atrophy, muscular dystrophy, myasthenia gravis and Lambert Eaton syndrome. The preferred muscular disease is muscular atrophy. Muscular atrophy is a decrease in the mass of the muscle which can progress partially or completely. Muscular dystrophy is any disease pertaining to structural defects in the muscle, mainly muscle weakness, defects in muscle proteins, and the death of muscle cells and tissue. More particularly, muscular dystrophy diseases are Duchenne, Becker, limb girdle, congenital, facioscapulohumeral, myotonic, oculopharyngeal, distal and Emery-Dreifuss muscular dystrophy.

A "disease of the peripheral nervous system" in the context of present invention is any disease characterized by lesions of the peripheral nerve cells. It is characterized by a deranged function and structure of peripheral motor, sensory, and/or autonomic neurons, involving either the entire neuron or selected levels. Peripheral neuropathic diseases include hereditary and acquired peripheral neuropathies. The preferred peripheral neuropathic disease in the context of present invention is acquired peripheral neuropathy. Symptomatic treatment acts on various ion channels and/or receptors of a sensory neuron, like a Na⁺ channel, a GABA mediated receptor and/or a proprioceptor, of the nerve cell to inhibit neuronal excitability. Drugs used with this intention include antiepileptics like gabapentin and carbamazepine, and local anaesthetics like lidocaine.

The term "osteoporosis" is referred to as understood in the art today, namely generally as a systemic skeletal disease, belonging to the group of degenerative bone diseases, characterized by low bone mass and deterioration of bone tissue, thus leading to an increased risk of fracture. Symptoms may be loss of height as a result of weakened spines, cramps in the legs at night, bone pain and tenderness, neck pain, discomfort in the neck other than from injury or trauma, persistent pain in the spine or muscles of the lower back, abdominal pain, tooth loss, rib pain, broken bones, spinal deformities, fatigue, periodontal disease, and/or brittle fingernails.

"Bone mineral density" (BMD) is a medical term referring to the amount of matter per cubic centimeter of bones. Bone mineral density can be measured employing conventional methods such as dual energy X-ray absorptiometry, which is the most common method to determine the bone mineral density.

The term "dual action bone agents" (DABAs) in the context of the present invention refers to agents that both increase the deposition of new bone by osteoblasts and reduce the resorption of bone by osteoclasts.

A "selective estrogen receptor modulator" (SERM) in the context of the present invention is a compound which acts on an estrogen receptor, preferably by binding with the estrogen receptor. Generally, a selective estrogen receptor modulator acts on an estrogen receptor selectively. Typically, its interaction with the estrogen receptor results in the inhibition of bone turnover and/or prevention of bone loss. A typical selective estrogen receptor modulator is raloxifene.

A "selective androgen receptor modulator" (SARM) in the context of present invention is in its broadest meaning an "androgen" (which may also be designated as "androgenic compound"). In the context of the present invention, this is a compound which acts on an androgen receptor, preferably by binding with the androgen receptor. Generally, a selective androgen receptor modulator acts on an androgen receptor selectively. Typically, its interaction with the androgen receptor results in an increase of cellular tissue, in particular of muscle tissue. Androgenic compounds are known to those skilled in the art. A typical androgen is testosterone.

A "cellular component" is any biological compound forming a vertebrate cell, e.g. a cellular structure (like a membrane or cellular compartment) or a biological compound (like a lipid, a protein or a nucleic acid). Preferably, it is a biological compound, more preferably a compound selected from the group consisting of ion channels, sensors and receptors, even more preferably a receptor, most preferably a membrane-bound receptor.

An "estrogen receptor" in the context of the present invention is a receptor whose activity is modulated by the hormone 17β-estradiol (estrogen). Generally, in present invention, 17β-estradiol activates the estrogen receptor. There are two types of estrogen receptors, an intracellular receptor belonging to the nuclear hormone family, and an estrogen G-protein coupled receptor. Both estrogen receptors are widely expressed in a broad range of cells, such as bone, kidney, brain, heart, lungs, intestinal mucosa, prostate, breast cancer, ovarian stroma, and endothelial cells, as well as in endometrium and hypothalamus. The estrogen receptor according to the present invention is preferably expressed in bone cells, more preferably in osteoblasts.

"Calcium sensing receptor" in the context of the present invention refers to a G-protein coupled receptor which senses extracellular levels of calcium ions. The calcium sensing receptor is expressed in a broad range of cell types in the osteoblasts, kidney, hematopoietic cells in bone marrow, and in the gastrointestinal mucosa. The calcium sensing receptor according to the present inventing is preferably expressed in bone cells, more preferably in osteoblasts.

An "androgen receptor" in the context of the present invention is a receptor whose activity is modulated by an androgen, typically by the hormone testosterone. Generally, in present invention, testosterone activates the androgen receptor. The androgen receptor according to the present invention is preferably expressed in cells of the neuromuscular unit, more preferably in muscle cells.

A "growth hormone receptor" (GHR) in the context of present invention is a transmembrane receptor for growth hormone. Generally, in present invention, growth hormone activates the growth hormone receptor by binding to the growth hormone receptor leading to receptor dimerization and the activation of an intra- and intercellular signal transduction pathway resulting in growth.

A "GABA receptor", also referred to as "GABA mediated receptor" in the context of present invention, is a receptor that responds to the neurotransmitter gamma-aminobutyric acid (GABA), the main inhibitory neurotransmitter in the vertebrate central nervous system. Generally, in present invention, GABA analogues, such as gabapentin, activate the GABA receptor by binding to the GABA receptor.

"Whole-body vibration" (WBV) in the context of the present invention refers to mechanical oscillations which are transferred to a vertebrate body as a whole, usually through a supporting system such as a platform.

Present invention pertains to the combined use of (A) mechanical vibration; and (B) a pharmaceutically active ingredient. Said combination is useful for the prevention, delay of progress, or treatment of a disease of the musculoskeletal system and/or a disease of the peripheral nervous system in a vertebrate, in particular of a degenerative bone disease like osteoporosis. Based on the particular effect of the combination of medical and vibration treatment, this method may allow lowering of the amount of pharmaceutically active ingredient administered to a vertebrate in need of treatment, resulting, e.g. in a reduction of the side-effects associated with conventional methods for treatment of a disease of the musculoskeletal system and/or a disease of the peripheral nervous system, and of polypharmacy.

The vertebrate to be treated by present invention is preferably a mammal, more preferably a primate, most preferably a human.

It was now found that the cellular response to the pharmaceutically active ingredient can be enhanced by performance of mechanical vibration, particularly whole-body vibration.

It is an object of the present invention to describe the preferred pharmaceutically active ingredients used in the combination according to present invention for the prevention, delay of progress, or treatment of a disease of the musculoskeletal system and/or a disease of peripheral nervous system in a vertebrate.

A further object of the invention is to describe a cell or a cellular component, in particular a receptor, whose responsiveness to the pharmaceutically active ingredient (B) is modified when applying the mechanical vibration element (A) of the combination therapy according to present invention.

It is a still further object of the invention to describe the mechanical vibration used in the combination according to present invention.

It is a still further object of the invention to describe the diseases of the musculoskeletal system and/or the diseases of the peripheral nervous system, which can be prevented, delayed, or treated employing the combination therapy according to the present invention.

### Mechanical Vibration

The mechanical vibration treatment, i.e. component (A) of the combination therapy according to present invention, may be any application of mechanical vibration to a vertebrate body. Generally, mechanical vibration refers to a mechanical oscillation about an equilibrium point.

The mechanical vibration used in the invention is typically repetitive, and periodic or random. A mechanical vibration treatment wherein the mechanical vibration is periodic or comprises blocks of periodic vibration is preferred. A block of such periodic vibration may have a duration from 1 to 120 sec, preferably from 5 to 30 sec, more preferably from 10 to 20 sec. The blocks of vibration are preferably interrupted by pauses, generally pauses with a duration from 1 to 300 sec, preferably from 10 to 60 sec, more preferably from 20 to 40 sec. A session typically comprises 1 to 50 blocks of vibration followed by a pause, preferably 5 to 30 blocks, more preferably 15 to 20 blocks.

The mechanical vibration used in the invention generally has a frequency of from 1 to 120 Hz, preferably from 20 to 60 Hz, more preferably from 35 to 45 Hz.

The intensity of vibration is determined by the operating frequency and the treatment time.

The mechanical vibration treatment of present invention is generally applied in 1 to 5 sessions a day, preferably 1 to 3 sessions, more preferably 1 to 2 sessions per day. In addition, the invention can be applied at one or more days per week, preferably at least at two days a week, more preferably at least at three days a week, even more preferably at least at four, five or six days a week or daily. Typically, it is applied at least at three days per week.

In order to achieve the desired preventive or therapeutic effect, the mechanical vibration treatment of present invention may be performed on the whole body of a vertebrate or on a part of said body, preferably on the whole body. If it is performed on a part of a vertebrate body, said part is typically a body part showing symptoms of the disease of the musculoskeletal system and/or the peripheral nervous system and/or being the origin of the disease of the musculoskeletal system and/or the peripheral nervous system. Typically, mechanical vibration treatment is applied to the limbs, the trunk and/or the spine.

When performing the mechanical vibration treatment on a body part, the treatment is preferably applied directly to said body part. When performing the mechanical vibration treatment on the whole body, the treatment is applied to the whole body.

The mechanical vibration treatment of present invention is performed on the body of a vertebrate. This implies performing said mechanical vibration treatment on one or more cells and/or one or more cellular components of said body. Preferably, said cells and/or cellular components are identical to the target cells and/or target cellular components of the pharmaceutically active ingredient which is element (B) of the combination therapy. Performing the mechanical vibration treatment on said cells and/or cellular components preferably enhances the responsiveness of said cells and/or cellular components to said API.

Said cells on which the mechanical vibration treatment is performed are preferably bone cells, cartilage cells, muscle cells or neural cells.

Said bone cells are selected from the group consisting of osteocytes, osteoblasts and osteoclasts, more preferably are osteoblasts.

Said cartilage cells are preferably selected from the group consisting of chondrocytes and subchondral osteocytes, more preferably are chondrocytes.

Said muscle cells are preferably selected from the group consisting of cells of the neuromuscular unit, more preferably are skeletal muscle cells.

Said neural cells are preferably selected from the group consisting of peripheral motor neurons, sensory neurons, and autonomic neurons, more preferably are sensory neurons.

Said cellular component on which the mechanical vibration treatment is performed may be any biological compound forming a vertebrate cell, preferably a cellular structure such as a membrane or a cellular compartment, or a biological compound such as a lipid, protein or nucleic acid. It is preferably selected from the group consisting of ion channels, sensors and receptors, more preferably it is a receptor. Said receptor may be located in a cell or on the surface of a cell, but is preferably part of a cell, like a membrane-bound receptor or an intracellular receptor. It is most preferably a membrane-bound receptor. Particularly preferred receptors are listed below in the section "Pharmaceutically active ingredient".

In the following, the mechanical vibration treatment of present invention will be described in more detail for whole-body vibration, which is the preferred mode of mechanical vibration treatment according to present invention. However, the technical parameters and instructions given in the following are also applicable for performing the invention using mechanical vibration treatment of only a part of a vertebrate body.

When performing present invention using whole-body vibration, preferably the entire vertebrate body is exposed to vibration. Whole-body vibration treatment in present invention is typically performed on a patient standing on a vibrating platform. This platform generates vertical sinusoidal vibrations, generated by the motors underneath the platform. These mechanical signals are transmitted to the body.

When performing the whole-body vibration according to the present invention, the intensity and the direction of the vibration used will determine the desired effect. The appropriate intensity and vibration direction in order to achieve the desired therapeutic effect will be dependent on the disorder being treated, the physical condition of the subject, and other factors that will be apparent to those skilled in the art or can be determined by standard techniques. The precise vibration treatment to be employed can be chosen according to the judgment of a medical practitioner and/or the subject's physical condition.

A further preferred aspect of present invention is the use of a vibration platform in the combination therapy. Any vibration platform capable of performing whole-body vibration can be used in the combination of the present invention, e.g. the commercially available whole-body vibration platforms used for osteoporosis therapy in the art. Different vibration platforms have different vibration characteristics. There are platforms which vibrate in three different directions: sideways, front and back and up and down (vertically). Which kind of vibration platform to choose in order to achieve maximum therapeutic effect depends mainly on the physical condition of the subject to be treated and on the vibration effect to be achieved.

In some instances the vibration direction is decisive for which body region is stimulated by the mechanical vibration. For example, when using vertical vibration, femoral or muscular structures of the lower limbs will benefit most, whereas horizontal vibration will have a stronger impact on spinal structures. Reciprocal vibration (combination of horizontal and vertical) will have the best effect since it affects both extremities as well as the trunk.

In embodiments (1) to (11) of present invention, the vibration direction may be selected from the group consisting of all available vibration directions, i.e. horizontal and vertical and any combination thereof. In a preferred aspect, the vibration direction is a combination of vertical and horizontal vibration (i.e. reciprocal vibration).

In one aspect of present invention, the mechanical vibration is a vertical vibration, i.e. up and down. The vertical vibration mode has the largest amplitude and is the most effective component in generating and inducing muscle contractions. Two types of movements are to be considered when using the vertical vibration mode: platforms mimicking that one foot is moving upwards and the other one at the same time downwards, and systems where the whole platform is mainly doing the same motion, respectively, both feet are moved upwards or downwards at the same time.

In another aspect of present invention, the mechanical vibration is a horizontal vibration, i.e. left to right or front to back, preferably front to back. The horizontal vibration mode has a strong impact on spinal structures which can increase BMD at the spinal level and help avoiding spinal fractures.

In a preferred aspect of embodiments (1) to (11), the vibration platform is a vibration platform with a peak to peak amplitude ranging from 1 mm to 5 mm, preferably ranging from 2 mm to 4 mm, more preferably a vibration platform with a peak to peak amplitude of about 2 mm.

In a preferred aspect of embodiments (1) to (11), the vertebrate in need of such a treatment is standing or sitting on the platform, more preferably the vertebrate is standing on the platform.

### Pharmaceutically Active Ingredient

The combination of the present invention comprises as a second element (B) the administration of a pharmaceutically active ingredient to the vertebrate. The pharmaceutically active ingredient for performing present invention may be any pharmaceutically active ingredient suitable for prevention, delay of progress, and/or therapy of a disease of the musculoskeletal system and/or a disease of the peripheral nervous system, particularly any pharmaceutically active ingredient suitable for prevention, delay of progress, and/or therapy of a disease of the musculoskeletal system and/or a disease of the peripheral nervous system which targets a cell and/or a cellular component (like a receptor) that can be stimulated by the mechanical vibration treatment (A).

In particular, the pharmaceutically active ingredient for use in the combination of the present invention may be any API which has been approved by a government authority, e.g. EMEA or FDA, for the treatment of diseases of the musculoskeletal system and/or diseases of the peripheral nervous system.

Preferably, any pharmaceutically active ingredient which modulates the activity of a cell (a "target cell" of the API in the context of present invention) and/or a cellular component (a "target cellular component"), particularly of a receptor, may be used in the present invention.

Said target cells are preferably bone cells, cartilage cells, muscle cells or peripheral neural cells. They are preferably identical to the cells whose responsiveness is enhanced by the mechanical vibration treatment (A) of present application.

Said target bone cells are selected from the group consisting of osteocytes, osteoblasts and osteoclasts, more preferably are osteoblasts.

Said target cartilage cells are preferably selected from the group consisting of chondrocytes and subchondral osteocytes, more preferably are chondrocytes.

Said target muscle cells are preferably selected from the group consisting of cells of the neuromuscular unit, more preferably are skeletal muscle cells.

Said target neural cells are preferably selected from the group consisting of peripheral motor neurons, sensory neurons, and autonomic neurons, more preferably are sensory neurons.

Said target cellular component may be any biological compound forming a vertebrate cell, preferably a cellular structure such as a membrane or a cellular compartment, or a biological compound such as a lipid, protein or nucleic acid. It is preferably selected from the group consisting of ion channels, sensors and receptors, more preferably it is a receptor. Said receptor may be located in a cell or on the surface of a cell, but is preferably part of a cell, like a membrane-bound receptor or an intracellular receptor. It is most preferably a membrane-bound receptor.

In a particularly preferred aspect of the invention, the pharmaceutically active ingredient modifies the activity of a receptor. Said receptor may be located in or on the surface of a cell, but is preferably part of a cell, like a membrane-bound receptor or an intracellular receptor. Membrane-bound receptors are especially preferred targets, e.g. G-protein coupled receptors.

In a first preferred aspect, said target receptor is a receptor selected from the group consisting of estrogen receptors and calcium sensing receptors. In said preferred aspect, the preferred disease is a disease of the musculoskeletal system, more preferably is a degenerative bone disease, in particular is osteoporosis.

Thus, in one preferred aspect of embodiments (1) to (3), (10) and (11), the pharmaceutically active ingredient is a compound which modulates the activity of an estrogen receptor, preferably by binding with said estrogen receptor. In a more preferred aspect, the pharmaceutically active ingredient modulates the activity of an estrogen receptor selected from the group consisting of intracellular estrogen receptors belonging to the nuclear hormone family and membrane-bound estrogen G-protein coupled receptors, most preferably of a membrane-bound estrogen G-protein coupled receptor.

Preferably, the target estrogen receptor according to the present invention is expressed in bone cells, more preferably in cells selected from the group consisting of osteoblasts and osteoclasts, even more preferably in osteoblasts.

In another preferred aspect of embodiments (1) to (3), (10) and (11), the pharmaceutically active ingredient is a compound which modulates the activity of a receptor which senses extracellular levels of calcium ions, in particular, of a calcium sensing receptor.

Preferably, the target calcium sensing receptor according to the present invention is expressed in bone cells, more preferably in cells selected from the group consisting of osteoblasts and osteoclasts, even more preferably in osteoblasts.

Representative pharmaceutically active ingredients which modulate the activity of an estrogen receptor or calcium sensing receptor belong to the group of selective estrogen receptor modulators and dual action bone agents, respectively. Selective estrogen receptor modulators and dual action bone agents are known to those skilled in the art, e.g. raloxifene, which belongs to the group of selective estrogen receptor modulators, strontium ranelate and peptides of the parathyroid hormone family, which belong to the group of dual action bone agents.

In a particularly preferred aspect, the pharmaceutically active ingredient for use in the combination of the present invention is raloxifene, lasofoxifene, arzoxifene and bazedoxifene or a pharmaceutically acceptable salt thereof, more preferably raloxifene, most preferably raloxifene-HCl.

In another particularly preferred aspect, the pharmaceutically active ingredient for use in the combination of the present invention is strontium ranelate or a pharmaceutically acceptable salt thereof, most preferably is strontium ranelate.

In another particularly preferred aspect, the pharmaceutically active ingredient for use in the combination of the present invention is a peptide of the parathyroid hormone family.

In a second preferred aspect, said target cell is a chondrocyte. In said preferred aspect, the preferred disease is a disease of the musculoskeletal system, preferably is a degenerative joint disease, in particular is osteoarthritis.

Thus, in one preferred aspect of embodiments (1), (4), (5), (10) and (11), the pharmaceutically active ingredient is a compound which modulates the activity of a chondrocyte. In a more preferred aspect, the pharmaceutically active ingredient modulates the activity of a glucose transporter of the chondrocyte.

In a particularly preferred aspect, the pharmaceutically active ingredient for use in the combination of the present invention is selected from the group consisting of glucosamine, chondroitin, diacerine, hyaluronic acid and its derivatives or a pharmaceutically acceptable salt of any of said compounds, more preferably is a glucosamine, most preferably is glucosamine sulfate.

In a third preferred aspect, said target cell or cellular component is a cell or cellular component of the neuromuscular unit. In said preferred aspect, the preferred disease is a disease of the musculoskeletal system, preferably is a muscular disease, in particular is muscular atrophy.

Thus, in one preferred aspect of embodiments (1), (6), (7), (10) and (11), the pharmaceutically active ingredient is a compound which modulates the activity of a muscle cell, preferably via an androgen receptor and/or a growth hormone receptor, or of a cellular component of such muscle cell, preferably an androgen receptor or a growth hormone receptor. In a more preferred aspect, the pharmaceutically active ingredient modulates the activity of an androgen receptor or a growth hormone receptor.

Representative pharmaceutically active ingredients which modulate the activity of an androgen receptor or growth hormone receptor belong to the group of androgenic compounds (like selective androgen receptor modulators) and growth hormones.

In a particularly preferred aspect, the pharmaceutically active ingredient for use in the combination of the present invention is testosterone or a pharmaceutically acceptable salt thereof, most preferably is testosterone. In another particularly preferred aspect, the pharmaceutically active ingredient for use in the combination of the present invention is a growth hormone or a pharmaceutically acceptable salt thereof, most preferably is a growth hormone.

In a fourth preferred aspect, said target cell or cellular component is a peripheral motor, sensory, and/or autonomic neuron or a cellular component thereof, preferably a sensory neuron or a receptor thereof, more preferably a receptor of a sensory neuron, most preferably a GABA mediated receptor. In said preferred aspect, the preferred peripheral neuropathic disease is acquired peripheral neuropathy.

Thus, in one preferred aspect of embodiments (1) and (8) to (11), the pharmaceutically active ingredient is a compound which modulates the activity of a neural cell, preferably via a Na⁺ channel, a GABA mediated receptor and/or a proprioceptor. In a more preferred aspect, the pharmaceutically active ingredient modulates the activity of a GABA mediated receptor.

In a particularly preferred aspect, the pharmaceutically active ingredient for use in the combination of the present invention is selected from the group consisting of gabapentin, carbamazepine, lidocaine and a pharmaceutically acceptable salt of any of said compounds, more preferably is gabapentin.

The dosage and single dose amount of the pharmaceutically active ingredient in the invention depends upon the desired therapeutic effect, on the route of administration and on the duration of the treatment. The dosage of the pharmaceutically active ingredient in the combination of the invention may be varied, depending on sex, age, stage of the disease, and body weight of the vertebrate in need of such treatment. The dosage of currently used medicaments in the treatment of the disease of the musculoskeletal system or the disease of the peripheral nervous system, respectively, will be known to a person skilled in the art (e.g. from the German Rote Liste: Arzneimittelverzeichnis für Deutschland, einschließlich EU-Zulassungen und bestimmter Medizinprodukte).

The pharmaceutically active ingredient for use in present invention may be administered by oral, parenteral (e.g., intramuscular, intraperitoneal, intravenous or subcutaneous injection, or implant), nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In a preferred aspect, the pharmaceutically active ingredient according to the present invention may be administered by oral or parenteral routes of administration, most preferred by an oral route of administration.

Typically, the dosage and single dose amount of the pharmaceutically active ingredient are as prescribed by a medical practitioner, the Package Information Leaflet and/or the manufacturer's instructions for administration when used in a conventional therapy of diseases of the musculoskeletal system and/or diseases of the peripheral nervous system without concomitant mechanical vibration treatment. However, due to the enhanced therapeutic effect caused by the mechanical vibration treatment, the dosage and/or single dose amount of the pharmaceutically active ingredient may be reduced in comparison to the conventional dosages (e.g. conventional dosages according to the Product Information Leaflet and/or the manufacturer's instructions). This is one particular advantage when performing the invention. In particular, the effective daily dose of the pharmaceutically active ingredient according to present invention can be lowered compared to the respective daily dose of same effectiveness in the absence of mechanical vibration treatment.

In a preferred aspect of embodiments (1) to (3), (10) and (11) of present invention, the amount of the pharmaceutically active ingredient raloxifene-HCl for use in the combination of the present invention is 60 mg/day. Due to the application of the mechanical vibration treatment, the amount may also be less than 60 mg/day, e.g. 50, 40, 30, or 20 mg/day.

In a further preferred aspect of embodiments (1) to (3), (10) and (11) of present invention, the amount of the pharmaceutically active ingredient strontium ranelate for use in the combination of the present invention is 2 g/day. Due to the application of the mechanical vibration treatment, the amount may also be less than 2000 mg/day, e.g. 1500, 1000, 500 or 200 mg/day.

In a further preferred aspect of embodiments (1), (4), (5), (10) and (11) of present invention, the amount of the pharmaceutically active ingredient glucosamine sulfate for use in the combination of the present invention is 1500 mg/day. Due to the application of the mechanical vibration treatment, the amount may also be less than 1500 mg/day, e.g. 1000, 750, 500, or 250 mg/day.

In a further preferred aspect of embodiments (1), (6), (7), (10) and (11) of present invention, the amount of the pharmaceutically active ingredient gabapentin for use in the combination of the present invention is in the range as defined by the Product Information Leaflet and/or the manufacturer's instructions (typically 800 mg/day). Due to the application of the mechanical vibration treatment, the amount may also be less than 800 mg/day, e.g. 600, 400, 200, or 100 mg/day.

In a further preferred aspect of embodiments (1), (8) to (11) of present invention, the amount of the pharmaceutically active ingredient testosterone for use in the combination of the present invention is in the range as defined by the Product Information Leaflet and/or the manufacturer's instructions. Due to the application of the mechanical vibration treatment, the amount may also be less than said defined range.

In a further preferred aspect of embodiments (1), (8) to (11) of present invention, the amount of the pharmaceutically active ingredient growth hormone for use in the combination of the present invention is in the range as defined by the Product Information Leaflet and/or the manufacturer's instructions. Due to the application of the mechanical vibration treatment, the amount may also be less than said defined range.

Optionally, dietary supplements may be administrated to the subject in addition to the pharmaceutically active ingredient of present invention. E.g., like in other therapies of diseases of the musculoskeletal system, it may be necessary to have supplements of calcium, magnesium and vitamin D if measured vitamin D levels are low or dietary calcium and magnesium intake is inadequate, to enhance the therapeutic effect of the pharmaceutically active ingredient. Therefore, in a preferred aspect of present invention, such supplements are given in the range as defined by the Product Information Leaflet and/or the manufacturer's instructions, more preferably supplemental calcium of 100 mg/day and/or vitamin D 500 IU/day. Due to the application of the mechanical vibration treatment, the amount of said supplements may also be less than said defined range. In particular, the amount of calcium may also be less than 100 mg/day, e.g. 75, 50, 25, or 10 mg/day, and the amount of vitamin D may also be less than 500 IU/day, e.g. 400, 300, 200, 100, or 50 IU/day.

In a preferred aspect of embodiment (2), the pharmaceutically active ingredient is raloxifene, particularly raloxifene-HCl, and the degenerative bone disease is osteoporosis.

In another preferred aspect of embodiment (2), the pharmaceutically active ingredient is strontium ranelate or a pharmaceutically acceptable salt thereof, particularly is strontium ranelate, and the degenerative bone disease is osteoporosis.

In a preferred aspect of embodiment (3), the pharmaceutically active ingredient is raloxifene, particularly raloxifene-HCl and the estrogen receptor is a membrane bound estrogen receptor.

In a preferred aspect of embodiment (3), the pharmaceutically active ingredient is strontium ranelate or a pharmaceutically acceptable salt thereof, particularly is strontium ranelate, and the calcium sensing receptor is a G-protein coupled calcium sensing receptor.

In a preferred aspect of embodiment (4), the pharmaceutically active ingredient is glucosamine, particularly glucosamine sulfate, and the degenerative joint disease is osteoarthritis.

In a preferred aspect of embodiment (5), the pharmaceutically active ingredient is glucosamine, particularly glucosamine sulfate, and the cellular component is a glucose transporter of a chondrocyte.

In a preferred aspect of embodiment (6), the pharmaceutically active ingredient is a growth hormone or a pharmaceutically acceptable salt thereof, and the muscular disease is muscle atrophy.

In another preferred aspect of embodiment (6), the pharmaceutically active ingredient is testosterone or a pharmaceutically acceptable salt thereof, and the muscular disease is muscle atrophy.

In a preferred aspect of embodiment (7), the pharmaceutically active ingredient is a growth hormone or a pharmaceutically acceptable salt thereof, and the receptor is a growth hormone receptor.

In another preferred aspect of embodiment (7), the pharmaceutically active ingredient is testosterone or a pharmaceutically acceptable salt thereof, and the receptor is an androgen receptor.

In a preferred aspect of embodiment (8), the pharmaceutically active ingredient is gabapentin or a pharmaceutically acceptable salt thereof, and the peripheral neuropathic disease is acquired peripheral neuropathy.

In a preferred aspect of embodiment (9), the pharmaceutically active ingredient is gabapentin or a pharmaceutically acceptable salt thereof, and the receptor is a GABA mediated receptor.

### Combination of Present Invention

Present invention provides a combination of the above two elements: (A) mechanical vibration treatment and (B) pharmaceutically active ingredient.

Said combination is used in the combination therapy of a disease of the musculoskeletal system and/or a disease of the peripheral nervous system by applying mechanical vibration treatment (A) to a vertebrate in addition to the administration of the pharmaceutically active ingredient (B). The mechanical vibration treatment typically enhances the responsiveness of the target cell and/or target cellular component of the API (B) to said API. Thus, the therapeutic or preventive effect of the pharmaceutically active ingredient is enhanced.

"Combination" and "combine" when used in context with the term "therapy" in present invention pertains to the concomitant application of mechanical vibration treatment over a period of treatment during which said vertebrate is also treated with the pharmaceutically active ingredient. The period of treatment wherein the mechanical vibration treatment is applied (in one or more sessions) may be shorter or longer than the period of time of administration of the pharmaceutically active ingredient, but will generally be identical to said latter period of treatment or start after the onset of the pharmaceutical treatment. This is due to the fact that typically the mechanical vibration treatment should start after a steady state level of the pharmaceutically active ingredient has been reached.

In the combination therapy of the invention the administration of the pharmaceutically active ingredient and performance of mechanical vibration treatment may occur separately or in conjunction. The administration or performance of one of the two elements (A) and (B) may be prior to, concurrent to, or subsequent to the administration or performance of the other element. Preferably, the pharmacologically active ingredient is administrated prior to a mechanical vibration treatment session.

In case the dosage form of the pharmaceutically active ingredient according to present invention results in a steady state level, performance of a mechanical vibration treatment session may occur at any point of time during the complete period of pharmacological treatment. In case the pharmaceutically active ingredient has a short half life, it is advised to administer said pharmaceutically active ingredient shortly before performing a mechanical vibration treatment session (e.g. not more than 2 hours prior to said session).

Preferably, the vertebrate diagnosed with a disease of the musculoskeletal system and/or a disease of the peripheral nervous system receives pharmacological treatment with the relevant pharmaceutical active ingredient, dose and dosage form. In addition, the vertebrate in need of such treatment receives mechanical vibration treatment, in particular whole-body vibration. The period of treatment with the mechanical vibration treatment sessions may continue for an unlimited period of time, e.g. days, weeks, months or years. Typically, the period of mechanical vibration treatment has a duration of at least a week, preferably from 1 week to 12 months, more preferably from 2 weeks to 6 months, even more preferably from 1 to 6 months, most preferably from 3 to 6 months. This encompasses any duration of time lying within said ranges, e.g. the period of mechanical vibration treatment used in the Example (about 4 weeks).

### Diseases of the musculoskeletal system

A preferred aim of present invention is the prevention, delay of progress, or treatment of a disease of the musculoskeletal system in a vertebrate, preferably of a degenerative bone disease, a degenerative joint disease, or a muscular disease.

In a preferred aspect of embodiments (1), (10) and (11), the disease of the musculoskeletal system is a degenerative bone disease (as in embodiment (2)).

Preferably, said degenerative bone disease is selected from the group consisting of osteochondritis, osteonecrosis, epiphysis, hyperostosis, osteitis deformans, osteoarthropathy, spinal diseases, and osteoporosis. In a more preferred aspect of embodiments (2), (10) and (11) the bone degenerative disease is osteoporosis.

In a further preferred aspect of embodiments (1), (10) and (11), the disease of the musculoskeletal system is a degenerative joint disease (as in embodiment (4)). Preferably, said degenerative joint disease is selected from the group consisting of ankylosis, arthralgia, arthritis, arthrogryposis, arthropathy, bursitis, chondromatosis, synovitis, and osteoarthritis. In a more preferred aspect of embodiments (4), (10) and (11) the degenerative joint disease is osteoarthritis.

In a further preferred aspect of embodiments (1), (10) and (11), the disease of the musculoskeletal system is a muscular disease (as in embodiment (6)).

Preferably, said muscular disease is selected from the group consisting of muscular atrophy, muscular dystrophy, myasthenia gravis and Lambert Eaton syndrome. In a more preferred aspect of embodiments (6), (10) and (11) the muscular disease is muscular atrophy.

### Diseases of the peripheral nervous system

A further aim of present invention is the prevention, delay of progress, or treatment of a disease of the peripheral nervous system in a vertebrate, preferably of a peripheral neuropathy.

In a preferred aspect of embodiments (1), (10) and (11), the disease of the peripheral nervous system is a peripheral neuropathic disease as in embodiment (8).

Preferably, said peripheral neuropathic disease is selected from the group consisting of acquired peripheral neuropathy and hereditary peripheral neuropathy. In a more preferred aspect of embodiments (8), (10) and (11) the peripheral neuropathic disease is acquired peripheral neuropathy.

### Effects of the combination according to present invention

When performing the present invention, the mechanical vibration treatment generally enhances the therapeutic effect of a pharmaceutically active ingredient by stimulating a target cell and/or a target cellular component (e.g. a receptor) which is a target of said pharmaceutically active ingredient. Said stimulation modulates (i.e. enhances) the responsiveness of said target to the API.

In the context of present invention, the effect of the pharmaceutically active ingredient is deemed to be "enhanced" if the effects of elements (A) and (B) are not just additive but synergistic. A synergistic effect means that the total effect exceeds the sum of the individual effects, e.g. if the effect of a drug intervention alone is 5% and the effect of vibration is 5%, a synergistic effect would be >10%. In particular aspects, such enhancement may be (i) the achievement of the same therapeutic effect whilst lowering the dosage and/or single dose amount of the pharmaceutically active ingredient compared to conventional dosage, (ii) improvement of the condition of the patient when using the conventional dosage in comparison to using the conventional dosage alone, (iii) a reduction of side-effects, (iv) a decrease in polypharmacy, and/or (v) a shortening of the period of treatment.

Typically, in order to achieve the desired therapeutic effect of the invention, said target cell and/or target cellular component must be responsive to the pharmacological intervention using the API and must be alive.

E.g., when performing the invention for preventing or treating osteoporosis, the preferred target cells are osteoblasts. Said cells are stimulated by the mechanical vibration treatment, which in turn enhances the responsiveness of the cell and thus the therapeutic effect of the pharmaceutically active ingredient which is administrated to the vertebrate, thus leading, e.g. to an increase in BMD.

A preferred aspect of the combination treatment according to the present invention is the stimulation of osteoblasts and/or estrogen receptors or calcium sensing receptors by the mechanical vibration treatment, thus enhancing the responsiveness of said target cells or target cellular components to an API. This is particularly useful for the treatment or prevention of a degenerative bone disease like osteoporosis.

A further preferred aspect of the combination treatment according to the present invention is the stimulation of chondrocytes by the mechanical vibration treatment, thus enhancing the responsiveness of said target cells to an API. This is particularly useful for the treatment or prevention of a degenerative joint disease like osteoarthritis.

A further preferred aspect of the combination treatment according to the present invention is the stimulation of the neuromuscular unit by the mechanical vibration treatment, thus enhancing the responsiveness of said target to an API. This is particularly useful for the treatment or prevention of a muscular disease like muscular atrophy.

A further preferred aspect of the combination treatment according to the present invention is the stimulation of ion channels, sensors and/or receptors of a neuron, preferably a peripheral, sensory and/or autonomic neuron by the mechanical vibration treatment, thus enhancing the responsiveness of said target peripheral, sensory and/or autonomic neuron to an API. This is particularly useful for the treatment or prevention of a disease of the peripheral nervous system like acquired peripheral neuropathy.

The treatment of diseases of the musculoskeletal system and/or diseases of the peripheral nervous system, according to present invention is particularly leading to an improvement of bone mineral density, joint cartilage, muscle properties and/or peripheral neuronal function. A treatment leading to an improvement of BMD is especially preferred.

For the treatment of a degenerative bone disease, particularly of osteoporosis, in a specific aspect of present invention, the mechanical vibration treatment causes compression and remodeling of the bone tissue, stimulating the osteoblasts, while reducing the responsiveness of the osteoclasts. Thus bone growth can be stimulated by the local elastic deformation of bone. A model of this mechanism is known as mechanostat (Frost H.M., Bone 1997, 20:385-391). An increase of bone mineral density over time results from repeated compression and remodeling of the bone tissue, combined with the increased pull on the bones by the muscles. When performing present invention, this typically has a positive effect on bone mineral density (e.g., increases BMD and/or reduces BMD reduction), and/or decreases pain associated with diseases of the musculoskeletal system.

In a particular aspect of present invention, the combined application of elements (A) and (B) increases BMD and/or reduces BMD reduction in comparison to administration of the pharmaceutically active ingredient without performing mechanical vibration treatment in combination with said administration. The BMD increase according to present invention is any measurable increase during the period of treatment in comparison with the sole administration of the API, preferably an increase by at least 1%, more preferably by at least 5%, even more preferably by at least 10% and more over 12 months period of treatment.

For the treatment of a degenerative joint disease, particularly of osteoarthritis, in a particular aspect of present invention, the combined application of elements (A) and (B) reduces or even prevents cartilage loss in comparison to administration of the pharmaceutically active ingredient without performing mechanical vibration treatment in combination with said administration. Said cartilage loss is measurable, e.g., by measuring the decrease of joint space narrowing (JSN). A JSN decrease according to present invention is any measurable decrease during the period of treatment in comparison with the sole administration of the API, preferably a decrease to less than 1 mm/year, even more preferably to less than 2 mm/year.

For the treatment of a muscular disease, particularly of muscle atrophy, in a particular aspect of present invention, the combined application of elements (A) and (B) increases muscular volume and muscle strength in comparison to administration of the pharmaceutically active ingredient without performing mechanical vibration treatment in combination with said administration. Said muscle strength is measurable, e.g., by evaluating circumference for muscle volume and completing a one-repetition maximum strength test for muscle strength. Muscular or muscle strength is referred to as the maximum force a muscle or muscle group can exert during a contraction. A muscle strength increase according to present invention is any measurable increase during the period of treatment in comparison with the sole administration of the API.

For the treatment of a peripheral neuropathic disease, particularly of acquired peripheral neuropathy, in a particular aspect of present invention, the combined application of elements (A) and (B) reduces neuropathic pain and/or increases proprioception in comparison to administration of the pharmaceutically active ingredient without performing mechanical vibration treatment in combination with said administration. Said pain is measurable, e.g., by pain questionnaires using a 100 mm visual analog scale. Proprioception is measurable by fluctuations recorded by three-dimensional Cranio-Corpo-Graphy (Banzer-Pfeifer-Vogt, Funktionsdiagnostik des Bewegungssystems in der Sportmedizin, Springer 2004). Pain reduction according to present invention is any measurable decrease in pain during the period of treatment in comparison with the sole administration of the API. Improved proprioception according to present invention is any measurable decrease in numbers of fluctuations during the period of treatment in comparison with the sole administration of the API.

Generally, and independently of the disease to be treated and the target cells and target cellular components, the mechanical signals caused by the mechanical vibration treatment stimulate sensory receptors in the vertebrate body, e.g. muscle spindles. This can lead to the activation of the alpha-motoneurons and initiates muscle contractions. Such contractions increase the speed of the blood flow through the body, allowing a better supply with nutrients. Thus, in said specific aspect, the combination therapy of present invention, in addition to enhancing the responsiveness of the target cells or target cellular components, improves the nutrient supply of body cells in comparison to the administration of the pharmaceutically active ingredient without concomitant mechanical vibration treatment.

Results of the combination therapy according to present invention can be measured by determining pharmacological (e.g. pharmacodynamic or pharmacokinetic) parameters characterizing the specific disease of the musculoskeletal system and/or disease of the peripheral nervous system to be treated. Such parameters may be BMD, JSN, muscle volume, muscle force, nerve conduction velocity (NCV), and parameters determinable, e.g., by electroencephalography (EGG), pain questionnaires, three-dimensional Cranio-Corpo-Graphy, blood and urine tests, and/or imaging tests (e.g., CT scan, MRI scan).

Bone mineral density is a typical pharmacodynamic parameter characterizing osteoporosis. To determine bone density and fracture risk for osteoporosis a bone mineral density test (BMD test) may be performed. There are several ways to measure bone mineral density available (Kanis J. A. et al., Osteoporos. Int., 2008, 19:399-428). Bone mineral density can be measured employing conventional methods such as dual energy X-ray absorptiometry, which is the most common method to determine the bone mineral density. Other suitable methods for bone mass determination besides dual energy X-ray absorptiometry are: peripheral dual energy X-ray absorptiometry, single energy X-ray absorptiometry, quantitative ultrasound, quantitative computed tomography, peripheral quantitative computed tomography, radiographic absorptiometry, dual photon absorptiometry, and single photon absorptiometry.

Joint space narrowing is a typical pharmacodynamic parameter characterizing osteoarthritis (Moskowitz et al. (Eds.) Osteoarthritis 4th Edition, 2007, Lippincott Williams & Wilkins). To measure joint space narrowing, imaging studies, such as X-ray, MRI or CT, are used to quantify joint damage by measuring the space that exists between the bones of a joint. Narrowing of the joint space indicates cartilage loss and worsening of osteoarthritis. The 5-point scale for grading radiographs, developed by Kellgren and Lawrence or quantitative evaluations in mm are used to quantify JSN.

Muscle strength is a typical pharmacodynamic parameter characterizing a muscular disease, such as muscular atrophy (Banzer-Pfeifer-Vogt, Funktionsdiagnostik des Bewegungssystems in der Sportmedizin, Springer 2004). The isotonic muscle strength is generally determined by completing a one-repetition maximum strength test. Muscular or muscle strength is referred as to the maximum force a muscle or muscle group can exert during a contraction.

There are many pharmacodynamic parameters, involving physical and neurological examination, characterizing a peripheral neuropathic disease, which are known to the person skilled in the art. Evaluating the symptoms like pain, sensory function using electrophysiological methods like nerve conduction velocity (NCV), electroencephalography (EGG), blood and urine tests, and imaging tests (e.g., CT scan, MRI scan) are just a few examples from a wide range of methods to determine the therapeutic effect of the treatment of a peripheral neuropathic disease (Rowland L. P. (Ed) Merrits Textbook of Neurology, 9th Edition, 1994, Williams & Wilkins).

Present invention offers the possibility to use lower single or combined doses of a pharmaceutically active ingredient in the prevention, delay or treatment of a disease of the musculoskeletal system and/or a disease of the peripheral nervous system, than the conventional single or combined doses. Consequently, a lower dosage of the pharmaceutically active ingredient may be used when performing present invention in comparison to conventional dosages. Therefore, present invention also contemplates a pharmaceutical composition which comprises a pharmaceutically active ingredient according to present invention in an amount which is below the minimum conventional therapeutically effective dose of said ingredient. The single or combined dose of the API according to present invention may be lowered by any percentage compared to conventional therapeutically effective dose of the pharmaceutically active ingredient, preferably by at least 10%, more preferably by at least 20%, even more preferably by at least 30% or any percentage inbetween said percentage values. In particular cases, it may be lowered by 50% or more %.

Present invention also offers the possibility to reduce the overall dosage of the pharmaceutically active ingredient in comparison to the conventional overall dosage, e.g. by increasing the period of time between each administration. This will also lead to a lower dosage of the pharmaceutically active ingredient to be used when performing present invention in comparison to conventional dosages.

The pharmaceutically active ingredient according to embodiment (11) may be any pharmaceutically active ingredient for use in the combination therapy according to present invention as described above. Similarly, the further preferred features of embodiment (11) are the preferred features as described in the previous sections.

The method according to embodiment (10) of present invention is a method for enhancing the therapeutic or preventive effect of a pharmaceutically active ingredient on a disease of the musculoskeletal system and/or a disease of the peripheral nervous system, comprising the application of mechanical vibration treatment to a vertebrate suffering from said disease. The method therefore relates to a therapeutic use.

In embodiment (10), the preferred features according to present invention as described in the previous sections specify the preferred features of the method.

The present invention is further described in more detail by reference to the following example(s). It should be understood that these examples are for illustrative purposes only and not to be construed as limiting the invention.

### Example

A human patient (No. 989) was diagnosed with osteoporosis and had a BMD of the spine as evaluated by Osteo CT of 83.8 on 9 March 2005. Patient received treatment with 60 mg of raloxifene daily. The BMD increased by 1.9% (85.4) when reevaluated at 26 January 2007. The patient continued treatment with 60 mg of raloxifene daily but received in addition whole body vibration therapy 4 times a week in 15 blocks of vibration at a frequency of 40 Hz and an amplitude of 2 mm for 15 sec interrupted by a pause of 30 sec from 21 June 2007 till 18 July 2007. The BMD as evaluated on 24 January 2008 was increased by 7.4% (91.7).

While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. It is intended, that the invention be defined by the scope of the claims which follow and that such claims be interpreted as broadly as is reasonable.

## Claims

1. A pharmaceutically active ingredient for use in a combination therapy in combination with mechanical vibration treatment for the prevention, delay of progress, or treatment of a disease of the musculoskeletal system and/or a disease of the peripheral nervous system in a vertebrate.

2. The pharmaceutically active ingredient according to claim 1, wherein the mechanical vibration treatment enhances the responsiveness of a cell and/or a cellular component to the pharmaceutically active ingredient.

3. The pharmaceutically active ingredient according to claim 2, wherein
(i) said cell is a cell selected from the group consisting of osteoblasts, chondrocytes, subchondral osteocytes, muscle cells, peripheral motor neurons, sensory neurons, and autonomic neurons; and/or
(ii) said cellular component is a cellular component selected from the group consisting of ion channels, sensors and receptors.

4. The pharmaceutically active ingredient according to any one of claims 1 to 3, wherein the mechanical vibration treatment is whole-body vibration.

5. The pharmaceutically active ingredient according to any one of claims 1 to 4, which modulates the activity of an estrogen receptor and/or calcium sensing receptor and which is preferably selected from the group consisting of selective estrogen receptor modulators and dual action bone agents.

6. The pharmaceutically active ingredient according to any one of claims 1 to 5, which is
(i) raloxifene or a pharmaceutically acceptable salt thereof, preferably raloxifene-HCl; or
(ii) strontium ranelate or a pharmaceutically acceptable salt thereof, preferably strontium ranelate.

7. The pharmaceutically active ingredient according to any one of claims 1 to 6, wherein the disease of the musculoskeletal system is a degenerative bone disease, preferably osteoporosis.

8. The pharmaceutically active ingredient according to any one of claims 1 to 4, which modulates the activity of a glucose transporter of a chondrocyte.

9. The pharmaceutically active ingredient according to any one of claims 1 to 4 and 8, which is selected from the group consisting of glucosamine, chondroitin, diacerine, hyaluronic acid and its derivatives, and a pharmaceutically acceptable salt thereof, more preferably is a glucosamine or a pharmaceutically acceptable salt thereof, most preferably is glucosamine sulfate.

10. The pharmaceutically active ingredient according to any one of claims 1 to 4, 8 and 9, wherein the disease of the musculoskeletal system is a degenerative joint disease, preferably osteoarthritis.

11. The pharmaceutically active ingredient according to any one of claims 1 to 4, which modulates the activity of a muscle cell, preferably the activity of an androgen receptor and/or a growth hormone receptor of said muscle cell.

12. The pharmaceutically active ingredient according to any one of claims 1 to 4 and 11, which is selected from the group consisting of androgens and growth hormones, preferably is
(i) testosterone or a pharmaceutically acceptable salt thereof; or
(ii) a growth hormone or a pharmaceutically acceptable salt thereof.

13. The pharmaceutically active ingredient according to any one of claims 1 to 4, 11 and 12, wherein the disease of the musculoskeletal system is a muscular disease, preferably muscular atrophy.

14. The pharmaceutically active ingredient according to any one of claims 1 to 4, which modulates the activity of a peripheral motor, sensory, and/or autonomic neuron, preferably of a sensory neuron.

15. The pharmaceutically active ingredient according to any one of claims 1 to 4 and 14, which is selected from the group consisting of antiepileptics, local anaestetics and other pharmaceutically active ingredients that modulate a Na⁺ channel, a GABA mediated receptor and/or a proprioceptor, preferably is gabapentin, carbamazepine, lidocaine or a pharmaceutically acceptable salt thereof, most preferably is gabapentin.

16. The pharmaceutically active ingredient according to any one of claims 1 to 4, 14 and 15, wherein the disease of the peripheral nervous system is a peripheral neuropathic disease, preferably acquired peripheral neuropathy.

17. The pharmaceutically active ingredient according to any one of claims 1 to 16, wherein the effective daily dose of said ingredient is lower than the respective daily dose of same effectiveness in the absence of mechanical vibration treatment.

18. A method for enhancing the therapeutic or preventive effect of a pharmaceutically active ingredient on a disease of the musculoskeletal system and/or a disease of the peripheral nervous system, comprising the application of mechanical vibration treatment to a vertebrate suffering from said disease.
